# EUROPÄISCHE PATENTSCHRIFT

(11) **EP 0 496 479 B1**
(45) Veröffentlichungstag und Bekanntmachung des Hinweises auf die Patenterteilung: **12.10.1994**
(21) Anmeldenummer: 92250002.0
(22) Anmeldetag: 06.01.1992
(51) Int. Cl.: A23L 1/30

(54) **NADH und NADPH als Energiesubstitute**
NADH and NADPH as energy-substitutes
NADPH et NADH comme substituts d'énergie

(30) Priorität: 24.01.1991 DE 4102240
(43) Veröffentlichungstag der Anmeldung: 29.07.1992
(73) Patentinhaber: Birkmayer, Jörg, Univ.-Prof. DDr., A-1090 Wien (AT)
(72) Erfinder: Birkmayer, Jörg, Univ.-Prof. DDr., A-1090 Wien (AT); Birkmayer, Walter, Prof. Dr., A-1090 Wien (AT)
(74) Vertreter: von Hellfeld, Axel, Dr. Dipl.-Phys.

(56) Entgegenhaltungen:
- EP-A- 0 107 161
- EP-A- 0 302 807
- PATENT ABSTRACTS OF JAPAN Band 10, Nr. 103 (C-340)(2160), 18. April 1986; & JP - A - 60234592 (TAKARA SHUZO K.K.) 21.11.1985

## Beschreibung

Die vorliegende Anmeldung betrifft neuartige Verwendung von Nikotinamid-adenin-dinukleotid in seiner reduzierten Form (NADH) und/oder Nikotinamid-adenin-dinukleotidphosphat in seiner reduzierten Form (NADPH) und/oder eines physiologisch verträglichen Salzes derselben sowie ein Lebens- oder Genußmittel für den menschlichen Verzehr oder ein Futtermittel für den tierischen Verzehr mit einem Gehalt an den genannten Substanzen.

Seit einigen Jahren sind insbesondere im Sportbereich verschiedenste Präparate zur Energiesteigerung in Mode gekommen, seien es die weitgehend verbotenen Anabolika zur Förderung des Muskelaufbaus oder relativ harmlose (aber auch weitgehend unwirksame) Präparate wie Sportgetränke, Schokoriegel, etc. .

Der vorliegenden Erfindung liegt die Aufgabe zugrunde, Präparate zur Verfügung zu stellen, die im menschlichen oder tierischen Körper als hochwirksame und völlig unschädliche Energiesubstitute wirken.

Erfindungsgemäß wird diese Aufgabe durch die Verwendung von Nikotinamid-adenin-dinukleotid in seiner reduzierten Form (NADH) und/oder Nikotinamid-adenin-dinukleotidphosphat in seiner reduzierten Form (NADPH) und/oder eines physiologisch verträglichen Salzes derselben zur Herstellung eines Mittels zur Energiesubstitution im menschlichen oder tierischen Körper gelöst. In einer bevorzugten Ausführungsform der Erfindung werden die genannten Substanzen einem Lebens- oder Genußmittel oder Futtermittel zugesetzt.

Alternativ dazu können die genannten Substanzen aber auch als einziger Wirkstoff oder Wirkstoffkomponente in einem Arzneimittel zur Verbesserung der Leistungsfähigkeit und/oder zur Retardierung von Abbau- und/oder Alterungsprozessen eingesetzt werden. Dabei besteht eine besonders bevorzugte Verwendung der genannten Substanzen als oder in einem Geriatrikum.

Besonders bevorzugt werden NADH und/oder NAPH und/oder das physiologisch verträgliche Salz derselben in einer Menge verabreicht, mit der dem menschlichen oder tierischen Körpern eine Einzeldosis von 0,01 bis 1,0 mg pro kg Körpergewicht zugeführt wird.

Weiterhin betrifft die Erfindung ein Lebens- oder Genußmittel für den menschlichen Verzehr, mit einem Gehalt an Nikotinamid-adenin-dinukleotid in seiner reduzierten Form (NADH) und/oder Nikotinamid-adenin-dinukleotidphosphat in seiner reduzierten Form (NADPH) und/oder einem physiologisch verträglich Salz derselben, wobei diese Substanzen bevorzugt in einer Menge enthalten sind, mit der dem menschlichen Körper 0,01 bis 1,0 mg pro Körpergewicht der besagte Substanzen pro Tages-Verzehreinheit zugeführt werden.

Schließlich betrifft die Erfindung noch ein Futtermittel für den tierischen Verzehr, mit einem Gehalt an Nikotinamid-adenin-nukleotid in seiner reduzierten Form (NADH) und/oder Nikotinamid-adenin-dinukleotidphosphat in seiner reduzierten Form (NADPH) und/oder einem physiologisch verträglichen Salz derselben, wobei besagte Substanzen bevorzugt in einer Menge enthalten sind, mit der den tierischen Körper 0,01 bis 1,0 mg pro kg Körpergewicht pro Tages-Verzehreinheit zugeführt werden.

Als "Tages-Verzehreinheit" soll diejenige Menge eines Lebens- oder Genußmittels oder Futtermittels verstanden werden, die von einem bestimmten Menschen oder einem bestimmten Tier üblicherweise pro Tag verzehrt wird. Da es sich bei NADH und NADPH um körpereigene Substanzen handelt, ist eine Erhöhung dieser Menge im Einzelfall allerdings unkritisch, da eventuelle Überdosierungen keinerlei schädliche Auswirkungen auf den Organismus haben.

Die Erfindung beruht auf der Erkenntnis, daß NADH und NADPH - neben anderen energiereichen Molekülen wie Adenintriphosphat (ATP) - die von den energieliefernden Nährstoffen, wie Kohlenhydraten, Fetten und Proteinen, freigesetzte Energie im Körper speichern und biosynthetischen Prozessen zugänglich machen.

Die wichtigste Rolle des NADH liegt in seiner treibenden Kraft für die Zellatmung. Unter Verwendung von Sauerstoff bildet NADH Wasser und drei Moleküle ATP nach der folgenden Formel:

NADH + H⁺ +1/2 O₂ + 3 Pᵢ + 3 ADP → NAD⁺ + 3 ATP + 4H₂O

Mit einem NADH-Molekül erhält man also drei ATP-Moleküle, die eine Energie von zusammen etwa 21 Kilokalorien darstellen. Diesen Vorgang nennt man oxydative Phosphorylierung. Die quantitative Bedeutung dieses Prozesses für den menschlichen Körper läßt sich durch ein einfaches Rechenbeispiel demonstrieren. Ein 70 kg schwerer Mensch verbraucht in Ruhe etwa 2800 Kilokalorien pro Tag. Diese Energiemenge kann durch Hydrolyse von etwa 384 mol ATP gewonnen werden, die 190 kg dieser Substanz entsprechen. Die im menschlichen Körper tatsächlich vorhandene ATP-Menge beträgt allerdings nur 50 g. Die restlichen 189,95 kg muß der Organismus daher jeden Tag selbst produzieren.

Durch Zufuhr von NADH bzw. NADPH kann man dem Organismus diese Arbeit wesentlich erleichtern, weil er dadurch größere Energiereserven besitzt. Außerdem können damit auch die vielen anderen von den beiden Coenzymen NADH und NADPH katalysierten Stoffwechselreaktionen stimuliert werden. Da ATP eine wichtige Rolle bei der Regulation des Gefäßtonus, der Muskelkontraktion, bei kardiovaskulären Funktionen, bei der Neurotransmission und bei der Blutplättchenaggregation spielt, die sämtliche zentrale Parameter für das Wohlbefinden und die Leistungsfähigkeit von Mensch und Tier sind, führt eine Zunahme von ATP-Molekülen durch Zufuhr von NADH oder NADPH insgesamt nicht nur zu einer Energiesubstitution, sondern insgesamt zu einer Verbesserung der Gesamtbefindlichkeit sowie einer Retardierung der Abbau- und/Alterungsprozesse.

Die Verwendung der erfindungsgemäßen Verbindungen ist dabei für den menschlichen und tierischen Organismus vollkommen unschädlich, da es sich hierbei um körpereigene Substanzen handelt. So wurden in klinischen Testreihen an Menschen bis zu 20fach höhere Konzentrationen als die angegebene Einzeldosis-Höchstgrenze bei Menschen als intravenöse Injektion verabreicht. Dabei konnten keinerlei Nebenwirkungen beobachtet werden. Darüberhinaus weiß man aus verschiedenen Untersuchungen, daß, wenn dem Organismus zuviel NADH oder NADPH zugeführt wird, es sofort zu NAD+ oder NADP+ oxidiert wird und dies eine reversible Gleichgewichtsreaktion ist.

Über weitere mögliche Wirkungsmechanismen von NADH bzw. NADPH, die zu den in den nachfolgenden Fallbeschreibungen noch näher beschriebenen weiteren positiven Auswirkungen auf den Organismus führen, gibt es derzeit noch kein klares Bild.

### Fallbeschreibungen

Fall 1: Tennisprofessional, 24 Jahre, männlich, erhält 5 mg NADH zweimal wöchentlich in Tablettenform. Nach 3 Wochen subjektive und objektive Steigerung der körperlichen Leistungsfähigkeit und der geistigen Fitness, geringere Müdigkeit nach dem Wettkampf, geringeres Schlafbedürfnis, erhöhte Wachheit und Aufmerksamkeit.

Fall 2: Manager, 49 Jahre, männlich, mit 14-Stunden-Arbeitstag, erhält einmal wöchentlich einen Schokoriegel mit einem Gehalt von 10 mg NADPH. Nach dreimaliger Einnahme deutliche Leistungssteigerung, geringere Ermüdbarkeit, kann Kaffee komplett weglassen, geringeres Schlafbedürfnis, erhöhte körperliche und geistige Aktivität nach dem Arbeitstag.

Fall 3: Künstler, 70 Jahre, männlich, arbeitsunlustig, inaktiv, schläft bis Mittag, Schlappheit, erhält zweimal wöchentlich (zum Verzehr an einem Tag) einen Liter eines Erfrischungsgetränkes mit einem Gehalt von 15 mg NADH. Nach 3 Wochen aktiv, energiegeladen, steht früh auf, arbeitet den ganzen Tag, gut gelaunt, fühlt sich körperlich und geistig frischer. Nach Absetzen der NADH-Applikation für 4 Wochen, Rückfall in den alten Zustand mit den alten Beschwerden. Neuerlicher Beginn mit Tabletten mit einem Gehalt von 5 mg NADPH zweimal wöchentlich. Nach einer Woche deutliche Verbesserung, nach 14 Tage Wiederherstellung des ursprünglichen aktiven Wohlbefindens.

Fall 4: Rennpferd, 6 Jahre, seit längerer Zeit keine Rennerfolge, Freß- und Bewegungsunlust. Nach sechswöchiger Verabreichung von Futtermittelpelles mit einem Gehalt von 0,5 mg pro Kilogramm Körpergewicht pro Tages-Verzehreinheit deutliche Steigerung der Freß- und Bewegungslust, deutlich aktiver und frischer, nach zehnwöchiger Behandlung erster Erfolg in einem Rennen.

Die in der vorstehenden Beschreibung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verwendung von Nikotinamid-adenin-dinukleotid in seiner reduzierten Form (NADH) und/oder Nikotinamid-adenindinukleotidphosphat in seiner reduzierten Form (NADPH) und/oder eines physiologisch verträglichen Salzes derselben zur Herstellung eines Mittels zur Energiesubstitution im menschlichen oder tierischen Körper.

2. Verwendung nach Anspruch 1 zur Herstellung eines Mittels als Zusatz zu einem Lebens- oder Genußmittel.

3. Verwendung nach Anspruch 1 zur Herstellung eines Mittels als Zusatz zu einem Futtermittel.

4. Verwendung nach Anspruch 1 zur Herstellung eines Mittels als Wirkstoff oder Wirkstoffkomponente in einem Arzneimittel zur Verbesserung der Leistungsfähigkeit und/oder zur Retardierung von Abbau und/oder Alterungsprozessen.

5. Verwendung nach Anspruch 4 als oder in einem Geriatrikum.

6. Verwendung nach einem der Ansprüche 2 bis 5, dadurch gekennzeichnet, daß NADH und/oder NADPH und/oder das physiologisch verträgliche Salz derselben in einer Menge verabreicht wird, mit der dem menschlichen oder tierischen Körper eine Einzeldosis von 0,01 bis 1,0 mg pro kg Körpergewicht zugeführt wird.

7. Lebens- oder Genußmittel für den menschlichen Verzehr mit einem Gehalt an Nikotinamid-adenin-dinukleotid in seiner reduzierten Form (NADH) und/oder Nikotinamid-adenin-dinukleotidphosphat in seiner reduzierten Form (NADPH) und/oder einem physiologisch verträglichen Salz derselben, dadurch gekennzeichnet, daß besagte Substanzen in einer Menge zugesetzt werden, mit der dem menschlichen Körper 0,01 bis 1,0 mg pro kg Körpergewicht der besagten Substanzen pro Tages-Verzehreinheit zugeführt werden.

8. Futtermittel für den tierischen Verzehr mit einem Gehalt an Nikotinamid-adenin-dinukleotid in seiner reduzierten Form (NADH) und/oder Nikotinamid-adenin-dinukleotidphosphat in seiner reduzierten Form (NADPH) und/oder einem physiologisch verträglichen Salz derselben, dadurch gekennzeichnet, daß besagte Substanzen in einer Menge zugesetzt werden, mit der dem tierischen Körper 0,01 bis 1,0 mg pro kg Körpergewicht pro Tages-Verzehreinheit zugeführt werden.

## Claims

1. Use of nicotinamide adenine dinucleotide (NADH) in its reduced form and/or of nicotinamide adenine dinucleotide phosphate (NADPH) in its reduced form, and/or of a physiologically compatible salt thereof to produce an agent for energy substitution in the human body or the animal body.

2. The use as claimed in claim 1 to produce an agent as an additive to a foodstuff or coffee, tea, alcoholic beverages or tobacco.

3. The use as claimed in claim 1 to produce an agent as an additive to a feed.

4. The use as claimed in claim 1 to produce an agent as an active substance or component of an active substance in a drug for improving performance and/or retarding decay and/or aging processes.

5. The use as claimed in claim 4 as a geriatric or in a geriatric.

6. The use as claimed in any one of claims 2 to 5, characterized in that NADH and/or NADPH and/or the physiologically compatible salt thereof is/are administered in an amount by which the human or animal body is supplied with an individual dose of from 0.01 to 1.0 mg per kilogram of body weight.

7. A food or coffee, tea, alcoholic beverage, or tobacco, for human consumption, including a content of nicotinamide adenine dinucleotide (NADH) in its reduced form and/or nicotinamide adenine dinucleotide phosphate (NADPH) in its reduced form, and/or a physiologically compatible salt thereof, **characterized** in that said substances are added in an amount by which the human body is supplied with from 0.01 to 1.0 mg per kilogram of bodyweight of said substances per daily consumption unit.

8. A feed for animal consumption, including a content of nicotinamide adenine dinucleotide (NADH) in its reduced form and/or nicotinamide adenine dinucleotide phosphate (NADPH) in its reduced form, and/or a physiologically compatible salt thereof, **characterized** in that said substances are added in an amount by which the animal body is supplied with from 0.01 to 1.0 mg per kilogram of bodyweight of said substances per daily consumption unit.

## Revendications

1. Utilisation de nicotinamide adénine dinucléotide sous sa forme réduite (NADH) et/ou de nicotinamide adénine dinucléotide phosphate sous sa forme réduite (NADPH) et/ou d'un sel physiologiquement toléré de ces derniers en vue de préparer un produit destiné à la substitution d'énergie dans l'organisme humain ou animal.

2. Utilisation selon la revendication 1 en vue de préparer un produit sous forme d'additif pour un aliment ou pour un produit de consommation de luxe.

3. Utilisation selon la revendication 1 en vue de préparer un produit sous forme d'additif pour un produit alimentaire pour animaux.

4. Utilisation selon la revendication 1 en vue de préparer un produit sous forme de principe actif ou de composant de principe actif dans un médicament pour améliorer l'endurance et/ou pour retarder les processus de dégradation et/ou de vieillissement.

5. Utilisation selon la revendication 4 sous forme de produit destiné aux personnes âgées ou dans un produit destiné aux personnes âgées.

6. Utilisation selon l'une quelconque des revendications 2 à 5, caractérisée en ce qu'on administre NADH et/ou NADPH et/ou le sel physiologiquement toléré de ces derniers en une quantité apportant à l'organisme humain ou animal une dose unique comprise entre 0,01 et 1,0 mg/kg de poids corporel.

7. Aliment ou produit de consommation de luxe destiné à la consommation humaine contenant du nicotinamide adénine dinucléotide sous sa forme réduite (NADH) et/ou du nicotinamide adénine dinucléotide phosphate sous sa forme réduite (NADPH) et/ou un sel physiologiquement toléré de ces derniers, caractérisé en ce qu'on utilise lesdites substances en une quantité apportant à l'organisme humain entre 0,01 et 1,0 mg/kg de poids corporel desdites substances par ration alimentaire journalière.

8. Produit alimentaire destiné à la consommation animale contenant du nicotinamide adénine dinucléotide sous sa forme réduite (NADH) et/ou du nicotinamide adénine dinucléotide phosphate sous sa forme réduite (NADPH) et/ou un sel physiologiquement toléré de ces derniers, caractérisé en ce qu'on utilise lesdites substances en une quantité apportant à l'organisme animal entre 0,01 et 1,0 mg/kg de poids corporel par ration alimentaire journalière.
